# EUROPÄISCHE PATENTANMELDUNG

(11) **EP 2 706 052 A2**
(43) Veröffentlichungstag der Anmeldung: **12.03.2014**
(21) Anmeldenummer: 13183402.0
(22) Anmeldetag: 06.09.2013
(51) Int. Cl.: C07C 41/09, C10L 1/02

(54) **Verfahren und Vorrichtung zur Verwendung von Methanol in einem Verbrennungsmotor, insbesondere einem Verbrennungsmotor mit Selbstzündung**

(30) Priorität: 07.09.2012 DE 102012017718
(71) Anmelder: Harzfeld, Georg, 18182 Rövershagen (DE)
(72) Erfinder: Harzfeld, Georg, 18182 Rövershagen (DE)
(74) Vertreter: Schulz, Manfred

(57) **Zusammenfassung**

Die Erfindung betrifft ein Verfahren und eine Vorrichtung zur Verwendung von Methanol in einem Verbrennungsmotor, insbesondere einem Verbrennungsmotor mit Selbstzündung.

Die Erfindung zeichnet sich dadurch aus,dass das Methanol in einer Vorrichtung zur internen Dehydratisierung erwärmt und über einen Katalysator geleitet wird, um Methanol zu Dimethyläther umzuwandeln, um diesen Dimethyläther der bzw. den Verbrennungskammer(n), vorzugsweise gemeinsam mit sonstigen Reaktionsprodukten direkt als Verbrennungskraftstoff zuzuführen.

## Beschreibung

Die Erfindung betrifft ein Verfahren und eine Vorrichtung zur Verwendung von Methanol in einem Verbrennungsmotor, insbesondere in einem Verbrennungsmotor mit Selbstzündung.

Im Stand der Technik ist es bekannt Dimethylether (DME) als Kraftstoff anstelle von Diesel einzusetzen. Es ist weiterhin die Herstellung von Dimethylether (DME) aus Methanol bzw. aus Synthesegas in einem Schritt über Methanol als Zwischenprodukt bekannt. Der Vorteil der Dimethylether(DME)-Anwendung liegt in der rußfreien Umsetzung des Kraftstoffs während des Verbrennungsvorganges im Verbrennungsmotor und der hohen Cetanzahl des DME im Vergleich zum Dieselkraftstoff.

Nachteilig wirkt sich die Tatsache aus, dass Dimethylether (DME) mit einem Siedepunkt von - 21°C unter atmosphärischem Druck bei Normaltemperatur gasförmig ist und daher ähnlich wie Flüssiggas Druckbehälter für Transport und Lagerung voraussetzt.

Ungeachtet dessen wird DME, insbesondere in China in steigendem Maße wegen seiner umweltfreundlichen Verwendung als Kraftstoff eingesetzt, da er rußfrei und NOₓ-arm verbrennt und im Motoraufbau erhebliche Einsparungen beim Partikelfilter und NOₓ-Katalysator gestattet.

Der Stand der Technik ist weiterhin dadurch gekennzeichnet, dass Methanol erfolgreich in Motoren mit Selbstzündung unter Zumischen von Zusatzstoffen als Kraftstoff eingesetzt wird. Es ist allerdings derzeit nicht möglich, Methanol ohne Zumischen von Zusatzstoffen als Hauptkraftstoff eines Verbrennungsmotors, z.B. in einem Kraftfahrzeug, sowie Dimethylether ohne entsprechende Flüssiggas-Druckbehälter einzusetzen.

Aufgabe der Erfindung ist es daher, eine Lösung vorzuschlagen, durch die Methanol am Verbrennungsmotor selbst bzw. am oder im Kraftfahrzeug so als Kraftstoff eingesetzt werden kann, dass er den hohen Ansprüchen hinsichtlich eines rußfreien und NOₓ-armen Einsatzes an Verbrennungsmotoren, insbesondere mit Selbstzündung genügt. Aufgabe der Erfindung ist es auch, Dimethylether ohne entsprechende Flüssiggas-Druckbehälter an einem Verbrennungsmotor bzw. einem Kraftfahrzeug mit Verbrennungsmotor einzusetzen.

Die Aufgabe der Erfindung wird gelöst durch ein Verfahren gemäß des Patentanspruchs 1 und eine Vorrichtung gemäß des Patentanspruchs 5.

In einer ersten Variante wird ein Verfahren zur Verwendung von Methanol in einem Verbrennungsmotor, insbesondere einem Verbrennungsmotor mit Selbstzündung vorgeschlagen, wobei das Methanol in einer Vorrichtung zur internen Dehydratisierung erwärmt und über einen Katalysator geleitet wird, um Methanol zu Dimethylether umzuwandeln, um diesen Dimethylether der bzw. den Verbrennungskammer/n, vorzugsweise gemeinsam mit sonstigen Reaktionsprodukten direkt als Verbrennungskraftstoff zuzuführen. Demzufolge wird durch das erfindungsgemäße Verfahren jetzt erreicht, dass das Dimethylether direkt am Verbrennungsmotor bzw. im Kraftfahrzeug hergestellt werden kann, ohne dass man hierzu die aufwendigen und sicherheitstechnisch komplizierten Flüssigkeitsgasdruckbehälter benötigt. Vielmehr wird in geschickter Art und Weise hier ausgenutzt, dass man Methanol zu Dimethylether umwandeln kann und zwar durch die Vorrichtung zur internen Dehydratisierung, die nun erfindungsgemäß am Verbrennungsmotor bzw. im Kraftfahrzeug - zum Beispiel an den Abgas führenden Leitungen - angeordnet wird. Die Vorteile dieser Erfindung sind natürlich enorm, da man aufgrund dessen, dass Methanol in ausreichendem Maße zur Verfügung steht, dieses sehr kostengünstige Produkt als Kraftstoff in einem Verbrennungsmotor, insbesondere einem Verbrennungsmotor mit Selbstzündung einsetzen kann.

Methanol kann bekanntermaßen aus CO₂-Abgasen von herkömmlichen Kraftwerken hergestellt werden und zwar beispielsweise unter Verwendung von überschüssigem Strom, der aus Windkraft- oder Solaranlagen oder dergleichen zur Verfügung steht und zwar dann, wenn in die Stromnetze z.B. bei Starkwindphasen zu viel Strom eingespeist wird oder in schwachen Abnahmephasen Überschuss entsteht. Somit hat man mehrere erfindungsgemäße Vorteile, nämlich zum einen die Verwendung von Methanol, das zu Dimethylether umgewandelt wird und dadurch nahezu rußfrei verbrennt. Im Kraftfahrzeug kann man daher beispielsweise Rußpartikelfilter einsparen. Zum anderen werden die Netze entlastet, indem man Methanol in sogenannten Hochstromphasen bzw. Schwachlastphasen, verbraucherseits gesehen, verwendet, um aus dem vorhandenen CO₂-Abgas dann Methanol zu erzeugen. Die erfindungsgemäßen Effekte liegen daher auf der Hand.

Erfindungsgemäß ist weiterhin vorgesehen, dass der Verbrennungsmotor zunächst mit herkömmlichem Kraftstoff gestartet wird, dann das Methanol unter Ausnutzung der Verbrennungswärme des Verbrennungsmotors erwärmt und über den Katalysator geleitet wird. Dies ist eine äußerst geschickte Variante, da man hier nicht unbedingt eine zusätzliche Heizung, in Form einer elektrischen Heizung oder in Form einer sogenannten Standheizung benötigt. Die Verbrennungswärme des zunächst mit Dieselkraftstoff gestarteten Motors reicht nach kurzer Startphase aus, um die notwendige Verbrennungswärme zur Verfügung zu stellen. In dem Moment, wo diese Wärme erreicht ist, kann man in der erfindungsgemäßen Dehydratisierungseinheit Methanol zu Dimethylether umwandeln und dann als Kraftstoff dem Verbrennungsmotor zuführen. Dazu kann man dann natürlich beispielsweise die Dieselkraftstoff-Zufuhr abstellen und man fährt komplett mit Methanol. Diese Variante der Erfindung ist am kostengünstigsten zu erhalten und wird daher bevorzugt.

Die Erfindung schließt jedoch auch ein, dass zum Start des Verbrennungsmotors Methanol als Brennstoff verwendet wird. Dabei ist es auch vorgesehen, dass das Methanol in die Vorrichtung zur internen Dehydratisierung gemeinsam mit den sonstigen Reaktionsprodukten als Gemisch von Dimethylether, Rest-Wasserdampf, nicht umgesetztem Methanol direkt der Verbrennung im Verbrennungsmotor zugeführt wird.

Als Katalysator wird ein für die Dehydratisierung von Methanol geeigneter Dehydratisierungskatalysator verwendet. Im Stand der Technik sind dazu entsprechende Katalysatoren bekannt, die hier sozusagen für einen neuen Verwendungszweck eingesetzt werden.

Ein Verfahren wie zuvor beschrieben ist auch dadurch gekennzeichnet, dass die Verdampfungswärme des Methanols (+ΔH293 = 37 kJ/mol) genutzt wird, um die Reaktionsenthalpie (-ΔH298) 23,4 kJ/mol) bei der adiabatischen Prozessführung weitgehend zu kompensieren, wobei eine entsprechende Produktführung, vorzugsweise von Gleichstrom hinsichtlich Abgas und Kraftstoff realisiert und so eine Erhöhung der Temperatur des Kraftstoffgemischs verhindert wird. Dabei ist es auch vorgesehen, dass die Dehydratisierung im Prinzip bei dem eingestellten Injektionsdruck stattfindet und keine wesentliche Druckerhöhung erfolgt bzw. eine solche nicht erforderlich ist. Des Weiteren ist es vorgesehen, dass durch die gewählte Prozessführung eine etwa gleichbleibende Reaktionstemperatur bei der Dehydratisierung eingehalten und damit eine günstige Gleichgewichtseinstellung der Dimethylether-Bildung erreicht wird. Von Vorteil ist es dabei, wenn die Dehydratisierungseinheit in dem Bereich des Abgasrohres des Motors installiert wird, in dem bei laufendem Motor eine Temperatur von 250°C - 300°C vorherrscht.

Eine Variante der Erfindung schlägt vor, dass die Dehydratation im Prinzip bei einem eingestellten Injektionsdruck stattfindet und eine wesentliche Druckerhöhung nicht erfolgt.

Dabei ist es auch von Vorteil, wenn eine Prozessführung gewählt wird, bei der eine etwa gleichbleibende Reaktionstemperatur bei der Dehydratisierung eingehalten und damit eine günstige Gleichgewichtseinstellung der Dimethylether - Bildung erreicht wird.

Erfindungsgemäß kann man an Stelle von sogenanntem AA-Methanol auch Cru-de-Methanol für die Dehydratisierung zu Dimethylether einsetzen. Dabei erfolgt die Verbrennung des verfahrensgemäß eingesetzten Methanols rußfrei und frei von karzinogenen Raktionsprodukten. Wie bereits eingangs erwähnt, ist es von besonderem Vorteil, als Kraftstoff vorrangig sogenanntes CER-Methanol einzusetzen, welches aus Wind-, Wasserstoff und Abgas-Kohlendioxid hergestellt wurde.

Erfindungsgemäß wird auch der Vorschlag unterbreitet, die im Abgassystem eines Dieselmotors nach dem Turboverdichter vorhandene Wärme zur Umsetzung von Methanol an einem geeigneten Katalysator zu Dimethyläther zu verwenden. Dazu wird das Abgas-Rohr mit einem weiteren Rohr umgeben und dicht verschweißt. In den Raum des so entstandenen Doppelrohrs wird der Dehydratisierungskatalysator eingefüllt.

Im Gleichstromverfahren zum Abgas wird das Methanol in das Doppelrohr mit hohem Druck eingespritzt bzw. eingedüst, verdampft und durch das Katalysatorbett geführt, so dass gegen Ende des Doppelrohrs die chemische Reaktion der Dehydratisierung des Methanols nach der Gleichung

2 CH₃OH → CH₃-O-CH₃+H₂O - ΔH = 23,4 kJ / mol

weitgehend abgeschlossen ist.

Trotz exothermer Dehydratisierung wird in geeigneter Weise durch Lamellen eine Abkühlung des Reaktionsgemisches von Dimethylether, Methanol und Wasser erreicht.

Dadurch erfolgt eine partielle Kondensation des Wasseranteils des Reaktionsgemisches. Das kondensierte Wasser wird ausgeschleust. Der Rest-Wasseranteil verbleibt dampfförmig im Reaktionsgemisch.

Die Dehydratisierung erfolgt beim Förderdruck der Hochdruck- Kraftstoffpumpe, die den Kraftstoff über das Katalysatorbett an der Wasserausschleusung bis zum Common Rail System der Kraftstoffeinspritzung (Eindüsung) in den Verbrennungsraum des Motors fördert.

Die Erfindung betrifft auch eine Vorrichtung zur internen Dehydratisierung von Methanol in einem Verbrennungsmotor, insbesondere mit Selbstzündung, die sich dadurch auszeichnet, dass die Vorrichtung einen Katalysatorraum umfasst, der eine Heizung aufweist und der Katalysatorraum eine Methanolzufuhr und eine Reaktionsproduktableitung aufweist, die mit der Brennstoffzufurh des Verbrennungsmotors verbunden bzw. verbindbar ist. Durch diese Dehydratisierungsvorrichtung ist es möglich, das Methanol direkt vor Ort in umweltfreundliches Dimethylether umzuwandeln, um dieses als Kraftstoff für den Verbrennungsmotor einzusetzen. Dabei ist als Heizung sowohl die Abgaswärme eines herkömmlichen Verbrennungsmotors, insbesondere eines Dieselmotors zu verstehen. Die Erfindung funktioniert allerdings auch mit einer Zusatzheizung, beispielsweise in Form einer elektrischen Heizung oder einer PKW-Zusatzheizung, die üblicherweise als sogenannte Standheizung bekannt ist.

Die bevorzugte Variante ist jedoch eine Lösung, bei welcher der Katalysatorraum in Art eines Doppelrohres das Abgasrohr des Verbrennungsmotors umgibt bzw. umhüllt und zwar in dem Bereich, in dem die zuvor bereits erwähnte gewünschte Temperatur von 250°C - 300°C vorherrscht.

Die durch die Dehydratisierung entstehende Abwärme wird durch eine Kühlung der Vorrichtung abgeleitet und zwar derart, dass immer eine ausreichende Prozesstemperatur vorhanden ist. Erfindungsgemäße wird hier vorgeschlagen, am Außenumfang der Vorrichtung Lamellen vorzusehen, welche die überschüssige Abwärme dann abführen.

Die Erfindung wird nachfolgend anhand von Ausführungsbeispielen weiter beschrieben.

Es zeigen:
- Fig. 1: Eine schematische Darstellung der Fließrichtung Abgas/Kraftstoff nach der Erfindung,
- Fig. 2: eine schematische Darstellung zur Installation einer Dehydratisierungseinheit nach der Erfindung.

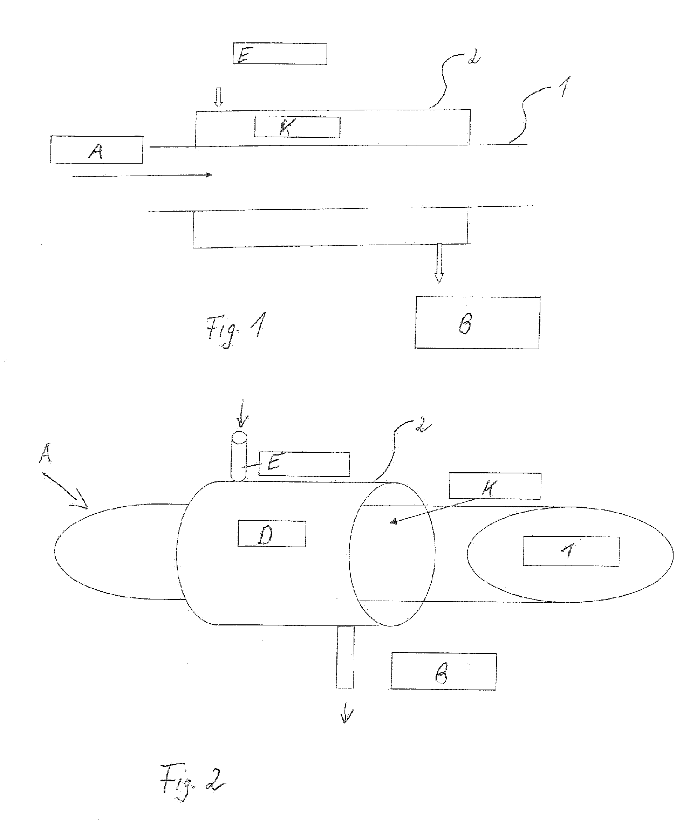

Fig. 1 zeigt eine schematische Darstellung der Fließrichtung Abgas/Kraftstoff nach der Erfindung. Dieses Fließbild zeigt, dass der Abgasstrom A gemäß dem Pfeil in dem Abgasrohr 1 geführt wird. Um dieses Abgasrohr 1 ist dann der Katalysatorraum K in Form eines Doppelrohres 2 vorgesehen. Dieses Doppelrohr 2 besitzt einen Methanoleingang E und am Ende der Dehydratisierungseinheit einen Reaktionsproduktausgang B. Dabei wird beispielsweise das Methanol so in den Katalysatorraum K eingeführt, dass es beispielsweise am Umfang des gesamten Katalysatorraumes K über dort angeordnete Düsen in den Katalysatorraum K eingespritzt bzw. eingedrückt wird. Damit wird eine ausreichende Verteilung des Methanols im Katalysatorraum K erreicht und so eine günstige Bedingung für den Dehydratisierungsprozess geschaffen. Mit dem Bezugszeichen K ist dabei der Katalysatorraum bezeichnet. Es bezeichnet den Methanoleingang bzw. die Methanolzuführung und B den Reaktionsproduktausgang.

In Fig. 2 ist eine schematische Darstellung zur Installation einer Dehydratisierungseinheit nach der Erfindung gezeigt. Hier sind ebenfalls gleiche Bezugszeichen verwendet, so dass auf eine erneute Vorstellung verzichtet werden kann. D bezeichnet die gesamte Dehydratisierungseinheit. Alle anderen Bezugszeichen entsprechen denen, die in Fig. 1 vorgestellt worden sind.

Die in den Figuren und im Ausführungsbeispiel angegebenen Merkmale stellen bevorzugte Varianten dar. Die Erfindung ist jedoch auf diese Merkmale bzw. Merkmalskombinationen nicht eingeschränkt. Vielmehr sind in der Beschreibung und den Patentansprüchen Merkmale angegeben, die für die Erfindung einen breiteren Schutz beanspruchen.

Die Erfindung wurde zuvor anhand von Ausführungsbeispieles beschrieben. Die jetzt und mit der Anmeldung später eingereichten Ansprüche sind Versuche zur Formulierung ohne Präjudiz für die Erzielung eines weitergehenden Schutzes.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die bislang nur in der Beschreibung offenbart wurden, können im Laufe des Verfahrens als von erfindungswesentlicher Bedeutung, zum Beispiel zur Abgrenzung vom Stand der Technik beansprucht werden.

## Patentansprüche

1. Verfahren zur Verwendung von Methanol in einem Verbrennungsmotor, insbesondere einem Verbrennungsmotor mit Selbstzündung, wobei das Methanol in einer Vorrichtung zur internen Dehydratisierung erwärmt und über einen Katalysator geleitet wird, um Methanol zu Dimethyläther umzuwandeln, um diesen Dimethyläther in eine bzw. mehrere Verbrennungskammer(n), vorzugsweise gemeinsam mit sonstigen Reaktionsprodukten direkt als Verbrennungskraftstoff zuzuführen.

2. Verfahren nach Anspruch 1, **dadurch gekennzeichnet, dass** der Verbrennungsmotor zunächst mit herkömmlichem Kraftstoff gestartet wird, dann das Methanol unter Ausnutzung der Verbrennungswärme des Verbrennungsmotors erwärmt und über den Katalysator geleitet wird.

3. Verfahren nach Anspruch 1, **dadurch gekennzeichnet, dass** zum Start des Verbrennungsmotors Methanol als Brennstoff verwendet wird.

4. Verfahren nach einem der vorhergehenden Ansprüche, **dadurch gekennzeichnet, dass** das Methanol in die Vorrichtung zur internen Dehydratisierung gemeinsam mit den sonstigen Reaktionsprodukten als Gemisch von Dimethylether, Rest-Wasserdampf, nicht umgesetztem Methanol direkt der Verbrennung im Verbrennungsmotor zugeführt wird.

5. Verfahren nach einem der vorhergehenden Ansprüche, **dadurch gekennzeichnet, dass** als Katalysator, ein für die katalytische Dehydratisierung geeigneter Dehydratisierungskatalysator verwendet wird.

6. Verfahren nach einem der vorhergehenden Ansprüche, **dadurch gekennzeichnet, dass**, die Verdampfungswärme des Methanols (+ΔH₂₉₃ = 37 kJ / mol) genutzt wird, um die Reaktionsenthalpie (- ΔH₂₉₈ = 23,4 kJ / mol) bei der adiabatischen Prozessführung weitgehend zu kompensieren, wobei eine entsprechende Produktführung, vorzugsweise von Gleichstrom hinsichtlich Abgas und Kraftstoff realisiert und eine Erhöhung der Temperatur des Kraftstoffgemisches verhindert wird.

7. Verfahren nach einem der vorhergehenden Ansprüche, **dadurch gekennzeichnet, dass** die Dehydratation im Prinzip bei einem eingestellten Injektionsdruck stattfindet und eine wesentliche Druckerhöhung nicht erfolgt.

8. Verfahren nach einem der vorhergehenden Ansprüche, **dadurch gekennzeichnet, dass** eine Prozessführung gewählt wird, bei der eine etwa gleichbleibende Reaktionstemperatur bei der Dehydratisierung eingehalten und damit eine günstige Gleichgewichtseinstellung der Dimethylether - Bildung erreicht wird.

9. Vorrichtung zur internen Dehydratisierung von Methanol in einem Verbrennungsmotor, insbesondere mit Selbstzündung und insbesondere zur Durchführung des Verfahrens nach einem der vorhergehenden Ansprüche 1 bis 8, wobei die Vorrichtung an dem Verbrennungsmotor bzw. an seinen medienführenden Zu- bzw. Ableitungen anordenbar ist, **dadurch gekennzeichnet, dass** die Vorrichtung einen Katalysatorraum umfasst, der eine Heizung aufweist und der Katalysatorraum eine Methanolzufuhr und eine Reaktionsproduktableitung aufweist, die mit der medienführende Zuleitung, wie z.B. die Brennstoffzuführung, des Verbrennungsmotors verbunden bzw. verbindbar ist.

10. Vorrichtung nach Anspruch 9, **dadurch gekennzeichnet, dass** der Katalysatorraum in Art eines Doppelrohres das Abgasrohr des Verbrennungsmotors umgibt bzw. umhüllt.

11. Vorrichtung nach der vorhergehenden Ansprüche 9 und 10, **dadurch gekennzeichnet, dass** eine Zusatzheizung vorgesehen ist, die insbesondere elektrisch beheizbar ist und/oder in Form einer PKW-Zusatzheizung (Standheizung) ausgebildet ist.

12. Vorrichtung nach einem der vorhergehenden Ansprüche 9 bis 11, **dadurch gekennzeichnet, dass** eine Kühlung der Vorrichtung, insbesondere in Form von außen am Umfang der Vorrichtung angeordneten Lamellen vorgesehen ist.
